(19) Europäisches Patentamt
European Patent Office
Office européen des brevets

(11) EP 2 113 252 A1

(12) EUROPEAN PATENT APPLICATION
published in accordance with Art. 153(4) EPC

(43) Date of publication:
**04.11.2009 Bulletin 2009/45**

(21) Application number: **07846011.0**

(22) Date of filing: **24.12.2007**

(51) Int Cl.:
***A61K 31/702*** *(2006.01)* ***A61K 9/08*** *(2006.01)*
***A61K 9/14*** *(2006.01)* ***A61P 29/00*** *(2006.01)*
***A61P 31/02*** *(2006.01)*

(86) International application number:
**PCT/CN2007/003757**

(87) International publication number:
**WO 2008/086698 (24.07.2008 Gazette 2008/30)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **28.12.2006 CN 200610148180**

(71) Applicants:
- **Shanghai Youseen New Medicine Exploition Co., Ltd.
Shanghai 201203 (CN)**
- **Yangtze River Pharmaceutical Group Co., Ltd.
Jiangsu 225321 (CN)**

(72) Inventors:
- **XUAN, Zhenyu
Shanghai 201203 (CN)**
- **WANG, Yong
Shanghai 201203 (CN)**
- **HUANG, Xiaochun
Shanghai 201203 (CN)**
- **LU, Hongguo
Jiangsu 225321 (CN)**
- **SUN, Tianjiang
Jiangsu 225321 (CN)**
- **CHEN, Guojun
Jiangsu 225321 (CN)**
- **TANG, Qiuling
Jiangsu 225321 (CN)**

(74) Representative: **Moir, Elizabeth et al
EIP
Fairfax House
15 Fulwood Place
GB-London WC1V 6HU (GB)**

# (54) A FORSYTHOSIDE INJECTION AND PREPARATION THEREOF

(57) The present invention discloses a Forsythiaside injection preparation and preparative method thereof. This injection preparation is prepared by Forsythiaside and pharmaceutical adjuvant.

Temperature (℃)
Time (h)
40
30
20
10
0
-10
-20
-30
-40
-50
0
2
4
6
8
10
12
14
16
18
20
22
24
26

Fig. 1

EP 2 113 252 A1

## Description

### Technical Field:

The present invention relates to the domain of pharmaceutical techniques. In particular, it relates to an injection preparation made of Forsythiaside, which is an active ingredient of *Forsythia Suspensa (Thunb.)* The present invention also relates to preparative methods of the Forsythiaside injection preparation.

### Background Technology:

Forsythiaside A is an active ingredient extracted from *Fructus Forsythiae, fruit of Forsythia Suspensa (Thunb.),* which belongs to the family Oleaceae and the genus Forsythia. In the past, Forsythin was used as the indicator of *Fructus Forsythiae* extracting techniques. However, with more research on active ingredients of *Fructus Forsythiae,* it has been found that the substance which is responsible for the antibacterial activities is Forsythiaside rather than Forsythin. *Fructus Forsythiae* contains a large amount of Forsythiaside A and Forsythiaside A plays a main role in exerting the antibacterial activities. However, it is hard for Forsythiaside A to exist on its own. Therefore the convention is to call Forsythiaside type of active ingredients containing Forsythiaside A "Forsythiaside". The structural formula of Forsythiaside (A) is as follows;

The Forsythiaside is a derivative of caffeic acid. There are an ester bond and a glucosidic bond in its molecular structure. It can be easily decomposed under the circumstances of acid, alkali and high temperature. And the caffeic acid, D-glucose, L-rhannose and first glycoside, which are generated as a result of decomposition, have a much weakened antibacterial activity. Since this problem was unknown, such medicines like Yinqiao Jiedu Pill and Yinqiao Jiedu Tablet can only be detected the ingredient of caffeic acid while the Forsythiaside (A) may have been hydrolyzed when processed.After much research, the inventors believe if people can extract Forsythiaside maximally and obtain highly purified Forsythiaside, then injection preparation made of such highly purified Forsythiaside must be able to supplement existing clinical drugs. In previous technical research, the inventors have acquired the techniques of extracting highly purified Forsythiaside. However, some pharmaceutical techniques involved in the Forsythiside injection preparation, such as the type or the dosage of the adjuvant, are not available in the existing technology. Meanwhile, the existing techniques reveal the instability of known Forsythiaside which is a serious obstacle in making a Forsythiaside injection preparation, but do not teach or suggest any solutions to this problem. Nevertheless, considering the medical efficacy of Forsythiaside, the inventors of this patent have carried out a large amount of scientific research on a beneficial quest into Forsythiaside injection preparations and have acquired unexpected results.

### Summary of the Invention:

In view of the above disadvantages in existing techniques, one of the problems to be solved by this invention is to provide a Forsythiaside injection preparation.

Another technical problem to be solved by this invention is to provide a preparative method of this Forsythiaside injection preparation.

This invention is achieved through the following technical schemes:

The Forsythiaside injection preparation mentioned in this invention is mainly prepared by Forsythiaside and pharmaceutical adjuvant with the proportion by weight of 1:(0-5), or preferably the proportion of 1:(0-3).

The Forsythiaside injection preparation in this invention includes three dosage forms: namely, lyophilized powder injection, water injection or infusion solution.

The Forsythiaside mentioned in this invention has a purity no less than 90% or suitable for use in injection. It can be extracted from the plant of Forsythia or can be bought or synthesized.

The pharmaceutical adjuvant used in the Forsythiaside lyophilized powder injection in present invention is called frame agent which contains one or a mixture of any two of the following: Mannitol, Glucose and Sorbitol randomly. The optimizing technical scheme is to add no frame agent into the Forsythiaside lyophilized powder injection. That means the proportion of Forsythiaside to frame agent is 1:0 by weight.

The Forsythiaside lyophilized powder injection in this invention is prepared through the following measures: mixing Forsythiaside and frame agent by their proportion by weight, and adding water for injection having a weight 10-50 times of that of the Forsythiaside to the mixture to dissolve the mixture and produce a solution, then adjusting the pH value of the solution to 3.0-6.0, putting the solution into containers after refined filtration and ultrafiltration. And the lyophilized injection is finally accomplished after freezing and drying according to Table1 below - the Lyophilizing Curve.

Table 1: Lyophilizing Curve of the Forsythiaside Lyophilized Powder Injection

| Temperature (°C) | Time (h) |
|---|---|
| 0 to -40 | 2 |
| -40 | 4 |
| -40 to -25 | 3 |
| -25 | 5 |
| -25 to 30 | 2 |
| -30 | 9 |

The pharmaceutical adjuvant used in the Forsythiaside water injection is called stabilizing agent which contains one or a mixing of any of the following: Disodium EDTA, Sodium -Calcium EDTA, Calcium EDTA, Vitamin C and Pyrosulfite.

The Forsythiaside water injection in this invention is prepared through the following measures: adding the stabilizing agent having a weight 0 to 5 times that of the Forsythiaside to water for injection to produce a solution, then stirring the solution until complete dissolution; adding activated carbon having a weight 0.5-0.05% of a total amount to the solution, stirring, filtering and decarbonizing; then adding Forsythiaside to the solution and making it fully dissolved, adjusting the pH value of the solution to 3.0-6.0; adding activated carbon having a weight 0.2-0.02% of the total amount to the solution, stirring the solution at room temperature, filtering, and decarbonizing the solution; adding water for injection to the solution to make up for the total amount; measuring the pH value and the contents of active ingredients of the solution. The solution is filtered repeatedly to become clear, put into containers, sterilized and packaged.

The pharmaceutical adjuvant used in the Forsythiaside infusion solution is called stabilizing agent which contains one or a mixture of any of the following: Disodium EDTA, Sodium -Calcium EDTA, Calcium EDTA, Vitamin C and Pyrosulfite.

The Forsythiaside infusion solution is prepared through the following measures: adding sodium chloride or glucose to water for injection to produce a solution and adding the stabilizing agent having a weight 0 to 5 times that of the Forthiaside to the solution, dissolving it fully; adding activated carbon having a weight 0.5-0.05% of a total amount, stirring, filtering and decarbonizing the solution; then adding Forsythiaside to the solution and stirring until it is fully dissolved; adjusting the pH value to 3.0-6.0; adding activated carbon having a weight 0.2-0.02% of the total amount to the solution, mixing, stirring the solution at room temperature, filtering, and decarbonizing the solution; adding water for injection to the solution to make up to the total amount, measuring the pH value of the solution and the contents of active ingredients in the solution; and filtering the solution repeatedly until it is clear, putting the solution into containers, sterilizing and packaging the solution.

In the preparation for the water injection and infusion solution mentioned above, the amount of activated carbon added is measured by percent weight per volume of the total amount. These above technical schemes applied in present invention have the following advantages: 1. It is a creation to use Forsythiaside as an active ingredient in injection preparation. Forsythiaside has always been a hot spot in research due to its antibacterial and anti-virus effects. What's more, Forsythiaside is considered as a potential detection index for medicine's constituents. However these had not been achieved. And current techniques provide no reports on using Forsythiaside as the active ingredient in injection preparations, let alone providing pharmaceutical techniques on Forsythiaside injection preparation, such as information on the type or the dosage of adjuvant. Furthermore, existing technology does not even suggest using Forsythiaside as

an active ingredient in injection preparation. After strict experimental screening, the present invention is able to provide the type and the dosage of the pharmaceutical adjuvant and the lyophilizing art of the lyophilized powder injection.

The present invention has also overcome the technical bias that normally lyophilized powder injection can take shape only if some protective adjuvant is added. The present invention shows that the Forsythiaside lyophilized powder injection can take good shape only by direct lyopilization and it is easy to lyophilize with or without adding adjuvant. It is also an unexpected result in techniques that the Forsythiaside lyophilized powder injection can meet the demand of the injection preparation without adding any pharmaceutical adjuvant. This not only simplifies the techniques, saves costs, but also avoids the safety problems introduced by adjuvant.

The present invention is invented according to the features of Forsythiaside. As the Forsythiaside is unstable at high temperature, the whole techniques applied in the present invention are under freezing conditions or at low temperatures to avoid the active ingredient, the Forsythiaside, being damaged. The lyophilized powder injection products have a loose texture and re-dissolve well after adding water to it. And the infusion solution and the water injection can also have a fine stability with the help of adjuvant.

The present invention can be implemented in industrial production. Meanwhile the injection preparation products of this invention have an excellent treatment effect, especially in the fields of fever relief and anti-inflammation. The products of the present invention also work on various bacteria and virus that undoubtedly means it offers promising marketing prospects and social benefits. So this invention has utility.

**Brief Description of the Accompanying Drawings:**

FIG.1 is a curve table depicting the lyophilizing artwork of the Forsythiaside lyophilized powder injection of this invention.

FIG.2 is a liquid chromatogram of the standard reference material of Forsythiaside in present invention.

FIG.3 is a liquid chromatogram of the sample product of the Forsythiaside lyophilized powder injection of this invention.

**Description of Preferred Embodiments:**

To further explain the present invention, the following practical examples are given, in which: example 1 to 9 are about lyophilized powder injection, example 10 to 14 are about water injection and example 15 to 18 are about infusion solution.

Example 1:

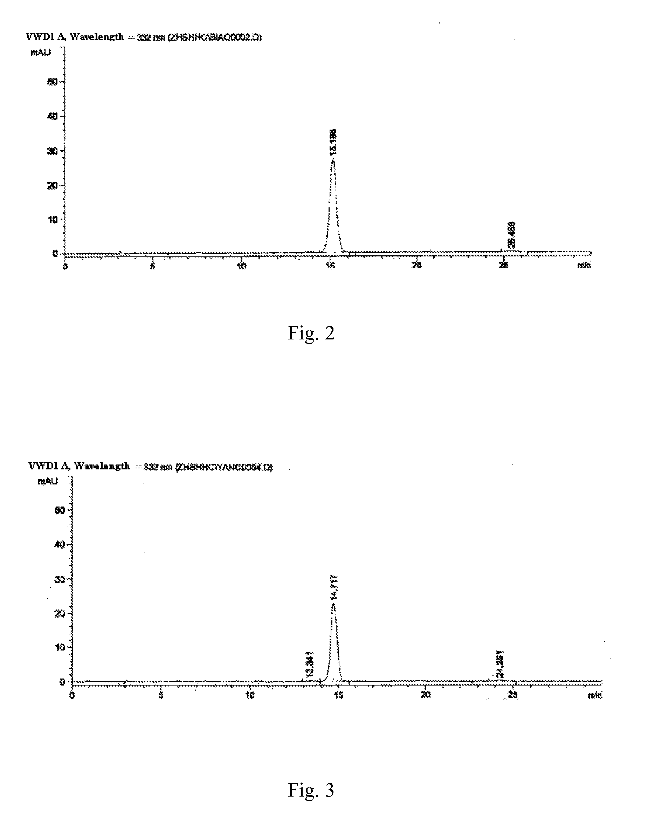

Taking 75g Forsythiaside, adding 1200ml water for injection, stirring and dissolving it, filtering the solution with 0.22μm microporous membrane for sterilization, then depyrogenating it with 8000 Dal ultrafiltration membrane. After the intermediate's assay is approved, filling the solution up to the fixed total volume with water for injection and filling the resulting solution separately into 1000 containers. Putting them into the refrigerator and freezing and drying them according to the lyophilizing curve as shown below. The liquid chromatogram of the standard reference material of Forsythiaside is as hown in FIG 2 .And the liquid chromatogram of the Forsythiaside lyophilized powder injection which is prepared using this method is shown in FIG 3.

Table 1: the lyophilizing curve is as follows:

| Temperature (°C) | Time (h) |
|---|---|
| 0 to -40 | 2 |
| -40 | 4 |
| -40 to -25 | 3 |
| -25 | 5 |
| -25 to 30 | 2 |
| -30 | 9 |

Example 2:

Taking 75g Forsythiaside and 75g Mannitol, respectively. Mixing them, adding 2000ml water for injection to them, then stirring and dissolving them. Adjusting the pH value to 5.5 with Acetic Acid-Sodium Acetate buffer salt, filtering the solution with 0.22μm microporous membrane for sterilization, then depyrogenating it with 8000 Dal ultrafiltration membrane. After the intermediate's assay is approved, filling the solution up to the fixed total volume with water for injection and filling the resulting solution separately into 1000 containers. Putting them into the refrigerator and freezing and drying them according to the lyophilizing curve before the preparation is accomplished. The lyophilizing curve applied in this example is the same as the one in Example 1.

Example 3:

Taking 50g Forsythiaside and 100g Glucose. Mixing them, adding 1000ml water for injection to them, then stirring and dissolving them. Adjusting the pH value to 5.0-6.0 with Citric Acid-Sodium Dihydrogen Phosphate buffet salt, filtering the solution with 0.22μm microporous membrane for sterilization, then depyrogenating it with 8000 Dal ultrafiltration membrane. After the intermediate's assay is approved, filling the solution up to the fixed total volume with water for injection and filling the resulting solution separately into 1000 containers. Putting them into the refrigerator and freezing and drying them according to the lyophilizing curve before the preparation is accomplished. The lyophilizing curve applied in this example is the same as the one in Example 1.

Example 4:

Taking 100g Forsythiaside, 75g Mannitol and 75g Glucose, respectively. Mixing them, adding 2500ml water for injection to them, then stirring and dissolving them. Adjusting the pH value to 3.0 with Acetate buffer salt, filtering the solution with 0.22μm microporous membrane for sterilization, then depyrogenating it with 8000 Dal ultrafiltration membrane. After the intermediate's assay is approved, filling the solution up to the fixed total volume with water for injection and filling the resulting solution separately into 1000 containers. Putting them into the refrigerator and freezing and drying them according to the lyophilizing curve before the preparation is accomplished. The lyophilizing curve applied in this example is the same as the one in Example 1.

Example 5:

Taking 100g Forsythiaside, 100g Mannitol and 100g Sorbitol separately. Mixing them, adding 3000ml water for injection to them, then stirring and dissolving them. Adjusting the pH value to 6.0 with Citric Acid-Sodium Dihydrogen Phosphate buffer salt, filtering the solution with 0.22μm microporous membrane for sterilization, then depyrogenating it with 8000 Dal ultrafiltration membrane. After the intermediate's assay is approved, filling the solution up to the fixed total volume with water for injection and filling the resulting solution separately into 1000 containers. Putting them into the refrigerator and freezing and drying them according to the lyophilizing curve before the preparation is accomplished. The lyophilizing curve applied in this example is the same as the one in Example 1.

Example 6:

Taking 150g Forsythiaside and 150g Glucose, respectively. Mixing them, adding 5000ml water for injection to them, then stirring and dissolving them. Adjusting the pH value to 4.5 with Acetic Acid-Sodium Acetate buffer salt, filtering the solution with 0.22μm microporous membrane for sterilization, then depyrogenating it with 8000 Dal ultrafiltration membrane. After the intermediate's assay is approved, filling the solution up to the fixed volume with water for injection and filling the end solution separately into 1000 containers. Putting them into the refrigerator and freezing and drying them according to the lyophilizing curve before the preparation is accomplished. The lyophilizing curve applied in this example is the same as the one in Example 1.

Example 7:

Taking 150g Forsythiaside, 150g Glucose and 150g Sorbitol separately. Mixing them, adding 6000ml water for injection to them, then stirring and dissolving them. Adjusting the pH value to 5.5 with Citric Acid-Sodium Dihydrogen Phosphate buffet salt, filtering the solution with 0.22μm microporous membrane for sterilization, then depyrogenating it with 8000 Dal ultrafiltration membrane. After the intermediate's assay is approved, filling the solution up to the fixed volume with water for injection and filling the resulting solution separately into 1000 containers. Putting them into the refrigerator and freezing and drying them according to the lyophilizing curve before the preparation is accomplished.

The lyophilizing curve applied in this example is the same as the one in Example 1.

Example 8:

Taking 200g Forsythiaside and 600g Sorbitol. Mixing them, adding 8000ml water for injection to them, then stirring and dissolving them. Adjusting the pH value to 6.0 with Citric Acid-Sodium Dihydrogen Phosphate buffet salt, filtering the solution with 0.22μm microporous membrane for sterilization, then depyrogenating it with 6000 Dal ultrafiltration membrane. After the intermediate's assay is approved, filling the solution up to the fixed volume with water for injection and filling the end solution separately into 4000 containers. Putting them into the refrigerator and freezing and drying them according to the lyophilizing curve before the preparation is accomplished. The lyophilizing curve applied in this example is the same as the one in Example 1.

Example 9:

Taking 75g Forsythiaside, 200g Mannitol and 175g Glucose. Mixing them, adding 3750ml water for injection to them, then stirring and dissolving them. Adjusting the pH value to 6.0 with Citric Acid-Sodium Dihydrogen Phosphate buffet salt, filtering the solution with 0.22μm microporous membrane for sterilization, then depyrogenating it with 8000 Dal ultrafiltration membrane. After the intermediate's assay is approved, filling the solution up to the fixed volume with water for injection and filling the end solution separately into 1000 containers. Putting them into the refrigerator and freezing and drying them according to the lyophilizing curve before the preparation is accomplished. The lyophilizing curve applied in this example is the same as the one in Example 1.

Example 10:

Adding 2.5g Sodium-Calcium EDTA to water for injection and dissolving it completely. Adding activated carbon which is 0.05% of a total amount of 2L, stirring, filtering and decarbonizing, then adding 75g Forsythiaside to the solution and making it fully dissolved. Adjusting the pH value to 3.0-6.0 with Citric Acid-Sodium Dihydrogen Phosphate buffet salt. Adding activated carbon again which is 0.02% of the total amount of 2L, stirring the solution at room temperature, filtering, decarbonizing and filling the solution up with water for injection to the total amount of 2L. Measuring the pH value and the contents of active ingredients. After the measurement results of the pH value and the content of Forsythiaside meet the above requirements, the solution should be filtered repeatedly to become clear, then filling it separately into 1000 containers, and sterilizing and packaging later.

Example 11:

Adding 2.5g Pyrosulfite to water for injection and dissolving it completely. Adding activated carbon which is 0.5% of a total amount of 5L, stirring, filtering and decarbonizing, then adding 150g Forsythiaside to the solution and making it fully dissolved. Regulating the pH value to 3.0-6.0 with Citric Acid-Sodium Dihydrogen Phosphate buffet salt. Adding activated carbon again which is 0.2% of the total amount of 5L, stirring the solution at room temperature, filtering, decarbonizing and filling the solution up with water for injection to the total amount of 5L. Measuring the pH value and the amount of Forsythiaside contained in the solution. After the measurement results of the pH value and the content of Forsythiaside meet the above requirements, the solution should be filtered repeatedly to become clear, then filling it separately into 1000 containers, and sterilizing and packaging later.

Example 12:

Adding 2.5g Disodium EDTA to water for injection and dissolving it completely. Adding activated carbon which equals 0.1% of a total amount of 10L, stirring, filtering and decarbonizing, then adding 150g Forsythiaside to the solution and making it fully dissolved. Regulating the pH value to 3.0-6.0 with Citric Acid-Sodium Dihydrogen Phosphate buffet salt. Adding activated carbon again which equals 0.1% of the total amount of 10L, stirring the solution at room temperature, filtering, decarbonizing and filling the solution up with water for injection to the total amount of 10L. Determining the pH value and the content of Forsythiaside in the solution. After the measurement results of the pH value and the content of Forsythiaside meet the above requirements, the solution should be filtered repeatedly to become clear, then filling it separately into 1000 containers, and perform sterilization and packaging later.

Example 13:

Adding 225g Vitamin C to water for injection and dissolving it completely. Adding activated carbon which equals

0.4% of the total amount of 5L, stirring, filtering and decarbonizing, then adding 75g Forsythiaside to the solution and making it fully dissolved. Regulating the pH value to 3.0-6.0 with Citric Acid-Sodium Dihydrogen Phosphate buffet salt. Adding activated carbon again which equals 0.05% of the total amount of 5L, stirring the solution at room temperature, filtering, decarbonizing and filling the solution up with water for injection to the total amount of 5L. Determining the pH value and the content of Forsythiaside of the solution. After the measurement results of the pH value and the content of Forsythiaside meet the above requirements, the solution should be filtered repeatedly to become clear, then filling it separately into 1000 containers, and sterilizing and packaging later.

Example 14:

Adding 375g Vitamin C to water for injection and dissolving it completely. Adding activated carbon which is 0.1% of the total amount of 10L, stirring, filtering and decarbonizing, then adding 75g Forsythiaside to the solution and making it fully dissolved. Regulating the pH value to 3.0-6.0 with Citric Acid-Sodium Dihydrogen Phosphate buffet salt. Adding activated carbon again which is 0.2% of the total amount of 10L. Stirring the solution at room temperature, filtering, decarbonizing and filling the solution up with water for injection to the total amount of 10L. Determining the pH value and the content of Forsythiaside. After the measurement results of the pH value and the content of Forsythiaside meet the above requirements, the solution should be filtered repeatedly till clear, then filling it separately into 1000 containers, and sterilizing and packaging later.

Example 15:

Adding 9.0g Sodium Chloride and 0.1g Sodium-Calcium EDTA to water for injection and dissolving them completely. Adding activated carbon which is 0.05% of a total amount of 1L, stirring, filtering and decarbonizing, then adding 0.6g Forsythiaside to the solution and making it fully dissolved. Regulating the pH value to 3.0-6.0 with Citric Acid-Sodium Dihydrogen Phosphate buffet salt. Adding activated carbon again which is 0.02% of the total amount of 1L, stirring the solution at room temperature, filtering, decarbonizing and filling the solution up with water for injection to the total amount of 1L. Determining the pH value and Forsythiaside content. After the measurement results of the pH value and the content of Forsythiaside meet the above requirements, the solution should be filtered repeatedly till clear, then filling it separately into 4 bottles, and sterilization and packaging later.

Example 16:

Adding 9.0g Sodium Chloride and 0.1g Pyrosulfite to water for injection and dissolving them completely to produce a solution. Adding activated carbon having a weight 0.3% of a total amount of 1L to the solution, stirring, filtering and decarbonizing the solution, then adding 0.3g Forsythiaside to the solution and making it fully dissolved. Adjusting the pH value to 3.0-6.0 with Citric Acid-Sodium Dihydrogen Phosphate buffet salt. Adding activated carbon having a weight 0.05% of the total amount of 1L to the solutin, stirring the solution at room temperature, filtering, decarbonizing and filling the solution up with water for injection to the total amount of 1L. Determining the pH value and the content of Forsythiaside in the solution. After the measurement results of the pH value and the content of Forsythiaside meet the above requirements, the solution should be filtered repeatedly until it is clear, then filling the solution separately into 4 ampoules, and sterilization and packaging later.

Example 17:

Adding 50g Glucose and 0.9g Vitamin C to water for injection and dissolving them completely to produce a solution. Adding activated carbon having a weight 0.05% of a total amount of 2L, stirring, filtering and decarbonizing the solution, then adding 0.3g Forsythiaside to the solution and making it fully dissolved. Adjusting the pH value of the solution to 3.0-6.0 with Citric Acid-Sodium Dihydrogen Phosphate buffet salt. Adding activated carbon having a weight 0.1% of the total amount of 2L, stirring the solution at room temperature, filtering, decarbonizing and filling the solution up with water for injection to the total amount of 2L. Determining the pH value and the content of Forsythiaside. After the measurement results of the pH value and the content of Forsythiaside meet the above requirements, the solution should be filtered repeatedly until it is clear, then filling it separately into 4 ampoules, and sterilization and packaging later.

Example 18:

Adding 100g Glucose and 1.5g Vitamin C to water for injection and dissolving them completely to produce a solution. Adding activated carbon which is 0.5% of a total amount of 1L, stirring, filtering and decarbonizing, then adding 0.3g Forsythiaside to the solution and making it fully dissolved. Adjusting the pH value to 3.0-6.0 with Citric Acid-Sodium

Dihydrogen Phosphate buffet salt. Adding activated carbon again which is 0.1% of the total amount of 1L, stirring the solution at room temperature, filtering, decarbonizing and filling the solution up with water for injection to the total amount of 1L. Determining the pH value and the content of Forsythiaside. After the measurement results of the pH value and the content of Forsythiaside meet the above requirements, the solution should be filtered repeatedly till it's clear, then filling it separately into 4 ampoules, and sterilization and packaging later.

The detailed explanations of the beneficial effects on fever relief and anti-inflammation of the Forsythiaside injection preparation in present invention will be discussed next. For the pharmacodynamical results of the Forsythiaside infusion solution, please refer to that of the Forsythiaside water injection.

1.1 pharmacodynamics experiment on fever-relief (fever given to SD rats by 2,4-Dinitrophenol injection)

Taking 48 SD rats, dividing them into 4 groups randomly, namely NS control group, SHL (ShuangHuangLian) control group, group of the Forsythiaside lyophilized powder injection, which is prepared according to one of the examples 1 to 9, group of the Forsythiaside water injection, which is prepared according to one of the examples 10 to 14. Before the experiment, measuring each rat's body temperature twice, and taking the mean values as their basal temperatures. A subcutaneous injection of 1ml/kg of 1.5 mg/ml extemporized 2,4-Dinitrophenol is administered to the backs of each rat, immediately after each rat was injected via the tail vein with 10ml/(kg·d) of the corresponding medicine of its group. 10ml/(kg·d) is 6 times the amount of clinical daily usage per person. Measuring and recording each animal's temperature at 30, 60, 90, 120, 180 and 240 minutes after taking the medication.

The experiment result shows: in comparison with the NS control group, group of the Forsythiaside lyophilized powder injection shows a significant antipyretic effect on SD rats' fever($p<0.05$)30 minutes later after taking the medication. 60-120 minutes after taking the medication, the group of the Forsythiaside water injection and the group of the Forsythiaside lyophilized powder injection show significant antipyretic effects in varying degrees on SD rats' fever ($p<0.05$,$p<0.01$) comparing to the NS control group. The SHL control group shows antipyretic effect on SD rats' fever comparing to the NS control group after taking the medication 30 min-180min($p<0.01$,$p<0.05$), while it shows no antipyretic effect comparing to the NS control group 240 minutes later after taking the medication. The result obtained is given set forth in Table 2.

Table2 : Temperature Changes of the Rats at Different Hours after Taking Medicine in the Experiment on Fever-relief (°C) ($\bar{x} \pm s$)

| Group | Mean Temperature before Medication | Temperature Changes at Different Hours after Medication | | | | | |
|---|---|---|---|---|---|---|---|
| | | 0.5h | 1h | 1.5h | 2h | 3h | 4h |
| NS Group | 38.19±0.25 | 0.77±0.13 | 1.47±0.26 | 1.45±0.26 | 1.37±0.22 | 1.12±0.23 | 0.68±0.25 |
| SHL Control Group | 38.18±0.28 | 0.51±0.26* | 0.81±0.28** | 0.84±0.28** | 0.64±0.32** | 0.59±0.21** | 0.47±0.20 |
| Forsythiaside Water Injection | 38.19±0.14 | 0.55±0.11 | 1.05±0.12** | 1.22±0.24* | 1.13±0.26* | 1.05±0.27 | 0.67±0.22 |
| Forsythiaside Lyophilized Powder Injection | 38.15±0.25 | 0.56±0.22* | 1.02±0.23** | 0.82±0.28** | 0.78±0.28** | 0.56±0.30** | 0.46±0.2 |

Note : In comparison with NS control group , * P<0.05 , **P<0.01

1.2 pharmacodynamics experiment on anti-inflammation

1.2.1 The Forsythiaside injection preparation's effects on the mice's swelling ears caused by Xylene

Taking 48 mice, dividing them into 4 groups randomly, the group categories are the same as those in the pharmacodynamics experiment on fever-relief mentioned above. Giving 10ml/(kg·d) medication by injection for 4 days, with 10ml/(kg·d) equaling 10 times the amount of clinical daily usage. 30 minutes after giving the last medication, smearing 50μl Xylene to each mouse's left ear and left the right ear as the blank control. Executing each mouse 15min after causing inflammation, and cutting both ears, punching down a piece of each ear at the same location on each ear with a hole puncher with a diameter of 7mm. Weighting these two ear pieces and calculating the swelling degree and the swelling inhibition ratio.

Formula:

$$\text{Swelling degree} = \text{Weight of the left ear slice} - \text{Weight of the right ear slice}$$

$$\text{Swelling inhibition ration} = \frac{\text{Swelling degree of the blank control group - Swelling of the medication group}}{\text{Swelling degree degree of the blank control group}} \times 100\%$$

The experiment shows: mice in the NS group have red and swollen left ears whose swelling degrees are up to 0.0145±0.0037g while the swelling degrees of group of each Forsythiaside injection preparation and SHL control group are all lower than that of the NS control group. And all of them have significant anti-inflammation effects on the mice's swellings ears in varying degrees comparing to the NS control group.($p<0.05,p<0.01$). The result obtained is set forth in Table 3.

Table 3: The Forsythiaside Injection Preparations' Effects on Mice's Swelling Ears Caused by Xylene

| Group | Number | Swelling Degree($\bar{x}$ ±s, g) | Swelling Inhibition Ration % |
|---|---|---|---|
| NS Group | 12 | 0.0145±0.0037 | - |
| SHL control group | 12 | 0.0099±0.0033** | 31.7 |
| the Forsythiaside Water Injection | 12 | 0.0109±0.0028* | 25.8 |
| the Forsythiaside Lyophilized Powder Injection | 12 | 0.0087±0.0039* | 40.0 |
| Note : In comparison with NS control group , * P<0.05 , **P<0.01 | | | |

1.2.2 The experiment of the Forsythiaside injection preparations' effects on vasopermeability

Taking 48 mice with a weight ranging from 18 to 22g, half of which are male mice and half female. The group categories are the same as those in the pharmacodynamics experiment on fever-relief mentioned above. 1 hour after taking the corresponding medicines, inject into tail vein 0.1ml of 0.5% Evans Blue Normal Saline per 10g of mouse weight. Giving an intraperitoneal injection with 0.20ml of 0.6% Acetic Acid per mouse, and 20 min later executing the mice by breaking the cervical vertebra. Then cutting the skin and muscle of the abdomen with a scissor, cleaning the abdominal cavity 3 times with a total of 6ml Normal Saline, sucking the solution with a pipette and combining the solutions. Adding Normal Saline to 10ml after the combination, centrifuging the combined solution at the speed of 3000 rpm for 15 minutes. Removing the clear solution on the top and having a colorimetric determination of the clear solution at

590nm, recording the OD value and conducting a statistical analysis.

The experiment result shows: in comparison with the NS control group, the group of the Forsythiaside water injection and the group of the Forsythiaside lyophilized powder injection have significant effects on vasopermeability ($p<0.05$), and the SHL control group also has a significant effect on vasopermeability comparing with the NS control group($p<0.05$).The result obtained is set forth in Table 4.

Table 4: the Forsythiaside Injection Preparations' Effects on the Improvements of the Vasopermeability in Mice's Abdominal Cavity after Having an Intraperitoneal Injection of Acetic Acid

| Group | Number | Weight | OD Value |
|---|---|---|---|
| NS Group | 10 | 21.98±1.14 | 0.0883±0.0208 |
| SHL control group | 10 | 22.05±1.07 | 0.0667±0.0155* |
| the Forsythiaside Water Injection | 10 | 22.03±1.20 | 0.0690±0.0140* |
| the Forsythiaside Lyophilized Powder Injection | 10 | 22.14±0.96 | 0.0685±0.0131* |
| Note : In comparison with NS control group , * P<0.05 , **P<0.01 | | | |

Next, we will take the lyophilized powder injection as an example to clearly explain reasons for choosing particular pharmaceutical adjuvant, the pH value in the preparation method and the lyophilizing curve of the Forsythiaside lyophilized powder injection. And we will also specify the stability of the Forsythiaside lyophilized powder injection by accelerated test.

2.1 Choice of the frame agents

The commonly used frame agents in the lyophilized powder injection are Mannitol, Glucose, Sorbitol, etc. We have designed an experiment for choosing a suitable type of frame agent. The result obtained is set forth in Table 5.

Table 5: Experiment on the Types of Frame Agents in the Forsythiaside Lyophilized Powder Injection

| Type of the Frame Agent | Volume of the Replenishment | Redissolution | Appearance of the Dissolubility Product |
|---|---|---|---|
| No frame agent | 2ml | Fine | Faint yellow lump, Fine relatively loose |
| No frame agent | 1.5ml | Fine | Faint yellow lump, Fine loose |
| 10% Mannitol | 1.5ml | Fine | Faint yellow lump, Fine relatively tight |
| 20% Mannitol | 1.5ml | Fine | Faint yellow lump, Fine relatively tight |
| 30% Mannitol | 1.5ml | Fine | Ivory and tight Fine lump |
| 50% Mannitol | 1.5ml | Fine | Ivory and tight Fine lump |
| 5%Glucose | 1.5ml | Fine | Shrink Fine |
| 10% Glucose | 1.5ml | Fine | Shrink Fine |
| 5%Sorbitol | 1.5ml | Fine | Ivory and tight Fine lump |

Table 6: Experiment on the Adding Amounts of Frame Agents in the Forsythiaside Lyophilized Powder Injection

| Forsythiaside | Adding Amounts of the Frame Agents(%) | | | | | | |
|---|---|---|---|---|---|---|---|
| | 0 | 50 | 100 | 200 | 300 | 400 | 500 |
| Shaping Property | General | General | Fine | Fine | Fine | General | Relatively poor |
| Dissolubility | Fine | Fine | Fine | Fine | Fine | General | General |

(continued)

| Forsythiaside | Adding Amounts of the Frame Agents(%) | | | | | | |
|---|---|---|---|---|---|---|---|
| | 0 | 50 | 100 | 200 | 300 | 400 | 500 |
| Clarity | Fine | Fine | Fine | General | General | Relatively poor | Relatively poor |

The research on the adding amounts of frame agents shows, when the proportion of Forsythiaside to frame agent is 1:(0-5) by weight, all the lyophilized powder can take shape well. However, the powder's shaping property, the dissolubility and the clarity after redissolution are even better when the weight proportion is 1:0-3. The present invention can obtain the powder which has a good shape, a fine dissolubility and a high clarity by freezing and drying directly without adding any frame agents. And it is also easy to lyophilize in this way.

2.2 Choice of the pH value

Taking appropriate amount of Forsythiaside, adding water to it for dissolution. Adjusting the pH value to 3.0, 3.5, 4.0, 4.5, 5.0, 5.5, 6.0 separately, then determining the peak area value of Forsythiaside at different pH value by HPLC to investigate the influence of different pH value on the content of Forsythiaside.

The result shows that: when the pH value is 4.5, Forsythiaside has the highest content and its content is not significantly varied when pH value is at 3.0-6.0. The result obtained is set forth in Table 7. So the pH value of the Forsythiaside lyophilized powder injection in present invention is controlled at 3.0-6.0.

Table 7: Result of the Experiment on Choice of The pH Value

| pH Value | 3.0 | 3.5 | 4.0 | 4.5 | 5.0 | 5.5 | 6.0 |
|---|---|---|---|---|---|---|---|
| Peak Area | 1410.0 | 1499.0 | 1519.8 | 1523.1 | 1494.5 | 1480.6 | 1423.5 |

2.3 Choice of the Lyophilized artwork curve

2.3.1 Setting freezing temperature

Generally, the temperature of the shelf should be 5°C-15°C lower than the eutectic point of the products. According to the above results which have been determined, the eutectic point of the product in this invention is -20°C, so the freezing temperature should be under -35°C and we set -40°C as the freezing temperature after selection.

2.3.2 Setting freezing time

In present invention each filling amount of the lyophilized powder injection is 1.5ml. After the experiment, we find that, it takes about 4h for the lyophilized powder injection in present invention to be frozen totally below the temperature of -40°C.

2.3.3 Sublimation velocity and drying time

Experiment shows: the primary drying phase needs a drying time of 5 hours at the temperature of -25°C, and the secondary drying phase needs another 9 hours when the temperature is no higher than 30°C, to meet the moisture content requirement in the lyophilized powder injection of the present invention.

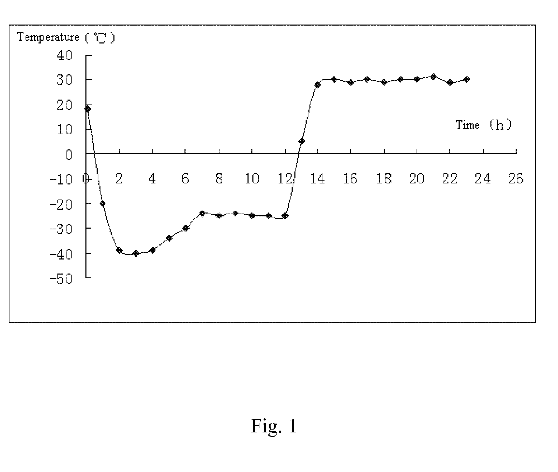

Prepare 3 batches of lyophilized powder injection according to the above screened optimal techniques, filling 1.5ml into each ampoule to be lyophilized. The preliminarily screened lyophilizing artwork curve is shown in Fig.1.

Lyophilize 10 batches of finished products according to the preliminarily screened lyophilizing artwork curve, and detect the lyophilization-related data e.g. moisture etc. The detection result is shown in Tab.8. The result shows all finished products meet the criterion and proves that the curve is feasible.

Tab.8: Observation on lyophilizing result for finished products

| Finished Products Lot Number | Description | Moisture Content (%) | Forsythiaside Content (%) |
|---|---|---|---|
| 2006060601 | meet the criterion | 3.24 | 92.00 |
| 2006060602 | meet the criterion | 3.68 | 92.05 |

(continued)

| Finished Products Lot Number | Description | Moisture Content (%) | Forsythiaside Content (%) |
|---|---|---|---|
| 2006060603 | meet the criterion | 3.71 | 91.89 |
| 2006060801 | meet the criterion | 3.15 | 91.93 |
| 2006060802 | meet the criterion | 3.57 | 92.08 |
| 2006060803 | meet the criterion | 3.81 | 91.92 |
| 2006060804 | meet the criterion | 3.09 | 92.31 |
| 2006061101 | meet the criterion | 3.60 | 91.71 |
| 2006061102 | meet the criterion | 3.25 | 92.15 |
| 2006061103 | meet the criterion | 2.91 | 92.07 |

2.4 Accelerated testing

Through accelerating pharmaceuticals' chemical or physical changes, we can investigate into the stability of a pharmaceutical preparation. And it also provides necessary information in the fields of prescription design, technique improvement, quality research, package improvement, transportation and storage. The conditions that need to be explored are as follows:

Package: The package which is intended to be sold on the market (Penicillin Bottle +Butyl Rubber Plugs)

Temperature: 40°C±2°C

Humidity: 75%±5%

Table 9: Result of the Accelerated Test

| Dosage Form | | Accelerated Test | | | | |
|---|---|---|---|---|---|---|
| | | 0 day | 15 days | 1 month | 2 months | 3 months |
| Lyophilized powder injection | Description | Solid powder with faint yellow color | Solid powder with faint yellow color | Solid powder with faint yellow color | Solid powder with faint yellow color | Solid powder with faint yellow color |
| | Content (%) | 93.0 | 92.9 | 92.8 | 92.5 | 92.1 |

The result of the accelerated test is shown in Table 9. From the test, we can see that while under the status of lyophilized powder, the Forsythiaside lyophilized powder injection can have a good stability and its description and content are not varied sufficiently which proves that it can comply with the requirement of injection preparation.

Next, we will take water injection and infusion solution as an example to clearly explain reasons for choosing particular pharmaceutical adjuvant, the pH value in the preparation method. And we will also specify the stability of the Forsythiaside lyophilized powder injection by accelerated test.

2.5 Choice of the Stabilizing Agents

The commonly used stabilizers in the water injection and infusion solution are Disodium EDTA, Sodium-Calcium EDTA, Calcium EDTA, Vitamin C and Pyrosulfite .We have designed an experiment for choosing a suitable type and amount of stabilizers. The result obtained is set forth in Table 10.

Table 10: Experiment on the Choice of Stabilizing Agents of the Forsythiaside Water Injection and Infusion Solution.

| Type of the stabilizer | Using Amount ( Principal Agent: Stabilizer ) | Product Appearance | Stability |
|---|---|---|---|
| Disodium EDTA | 1 : 0.2 | Clear liquid with faint yellow color | Fine |
| | 1 : 0.5 | Clear liquid with faint yellow color | Good |
| Sodium -Calcium EDTA | 1 : 0.5 | Clear liquid with faint yellow color | Fine |
| | 1 : 1 | Clear liquid with faint yellow color | Fine |
| Calcium EDTA | 1 : 0.3 | Clear liquid with faint yellow color | Fine |
| | 1 : 0.5 | Clear liquid with faint yellow color | Fine |
| Vitamin C | 1 : 2 | Clear liquid with faint yellow color | Good |
| | 1 : 5 | Clear liquid with faint yellow color | Fine |
| Pyrosulfite | 1 : 1 | Clear liquid with faint yellow color | Fine |
| | 1 : 3 | Clear liquid with faint yellow color | Good |

The result shows that: the water injection and the infusion solution can maintain stable when the proportion of Forsythiaside to stabilizing agent is 1: 0-5 by weight, and they can maintain stable better when the proportion is 1:0-3

2.6 Choice of the pH value

Choice of the pH value of the water injection and the infusion solution is the same as that of the lyophilized powder injection. The pH value of the lyophilized powder injection in present invention is controlled at 3.0-6.0.

2.7 Accelerated testing

Through accelerating medicine's chemical or physical changes, we can investigate into the stability of a pharmaceutical preparation. And it also provides necessary information in the fields of prescription design, technique improvement, quality research, package improvement, transportation and storage. The conditions that need to be explored are as follows:

Package: The package which is intended to be sold on the market (ampoule or infusion bottle)

Temperature: 40°C±2°C

Humidity: 75%±5%

Table 11: Result of the Accelerated Test

| Dosage Form | | Accelerated Test | | | | |
|---|---|---|---|---|---|---|
| | | 0 day | 15 days | 1 month | 2 months | 3 months |
| Water Injection | Description | Clear liquid with faint yellow color | Clear liquid with faint yellow color | Clear liquid with faint yellow color | Clear liquid with faint yellow color | Clear liquid with faint yellow color |
| | Content (%) | 92.6 | 92.1 | 92.0 | 92.2 | 91.8 |

(continued)

| Dosage Form | | Accelerated Test | | | | |
|---|---|---|---|---|---|---|
| | | 0 day | 15 days | 1 month | 2 months | 3 months |
| Infusion Solution | Description | Clear liquid with faint yellow color | Clear liquid with faint yellow color | Clear liquid with faint yellow color | Clear liquid with faint yellow color | Clear liquid with faint yellow color |
| | Content (%) | 92.2 | 92.0 | 91.9 | 92.0 | 91.5 |

The result of the accelerated test is shown in Table 11. From the test, we can see that while under the status of water injection and infusion solution, the Forsythiaside injection can have a good stability and its description and content are not varied sufficiently which proves that it can comply with the requirement of injection preparation.

## Claims

1. A Forsythiaside injection preparation, prepared by Forsythiaside and a pharmaceutical adjuvant with a proportion by weight of 1: (0-5).

2. The Forsythiaside injection preparation according to Claim 1, wherein the proportion by weight of the Forsythiaside to the pharmaceutical adjuvant is 1: (0-3).

3. The Forsythiaside injection preparation according to Claim 1 or Claim 2, wherein the injection preparation includes lyophilized powder injection, water injection or infusion solution.

4. The Forsythiaside injection preparation according to Claim 3, wherein the pharmaceutical adjuvant for the lyophilized powder injection is one or a mixture of any two of the following: Mannitol, Glucose and Sorbitol.

5. The Forsythiaside injection preparation according to Claim 4, wherein the proportion by weight of the Forsythiaside to the pharmaceutical adjuvant is 1: 0.

6. A preparative method for the Forsythiaside injection preparation according to Claim 4 or Claim 5, comprising the steps of:

   mixing the Forsythiaside and the pharmaceutical adjuvant according to their proportion by weight;
   adding water for injection having a weight 10-50 times that of the Forsythiaside to dissolve the mixture and produce a solution;
   adjusting the pH value of the solution to 3.0-6.0;
   performing refined filtration and ultrafiltration of the solution before putting the solution into containers; and lyophilizing the solution in the containers according to the following lyophilizing curve.

| Temperature (°C) | Time (h) |
|---|---|
| 0 to -40 | 2 |
| -40 | 4 |
| -40 to -25 | 3 |
| -25 | 5 |
| -25 to 30 | 2 |
| -30 | 9 |

7. The Forsythiaside injection preparation according to Claim 3, wherein the pharmaceutical adjuvant for the Forsythiaside water injection is one or a mixture of any of the following: Disodium EDTA, Sodium-Calcium EDTA, Calcium EDTA, Vitamin C and Pyrosulfite.

**8.** A preparative method for the Forsythiaside injection preparation according to Claim 7, comprising the steps of:

adding the pharmaceutical adjuvant having a weight 0-5 times that of the Forsythiaside into water for injection, and stirring until it dissolves completely to produce a solution;
adding activated carbon having a weight 0.5-0.05% of a total amount to the solution, stirring, filtering and decarbonizing the solution;
adding to the solution and fully dissolving the Forsythiaside , and adjusting the pH value of the solution to 3.0-6.0;
adding activated carbon having a weight 0.2-0.02% of the total amount to the solution, stirring at room temperature, filtering, and decarbonizing the solution;
adding water for injection into the solution until the total amount is reached, measuring the pH value of the solution and the contents of active ingredients of the solution; and
after being qualified, filtering the solution repeatedly until the solution is clear, before putting the solution into containers, sterilizing and packaging the solution.

**9.** The Forsythiaside injection preparation according to Claim 3, wherein the pharmaceutical adjuvant for the Forsythiaside infusion solution is one or a mixture of any of the following: Disodium EDTA, Sodium-Calcium EDTA, Calcium EDTA, Vitamin C and Pyrosulfite.

**10.** A preparative method for the Forsythiaside injection preparation according to Claim 9, comprising the steps of:

adding Sodium Chloride or Glucose to water for injection to produce a solution;
adding the pharmaceutical adjuvant having a weight 0-5 times that of the Forsythiaside to the solution and stirring the solution until it dissolves completely;
adding activated carbon having a weight 0.5-0.05% of a total amount to the solution, stirring, filtering and decarbonizing the solution;
adding the Forsythiaside to the solution, stirring until it is fully dissolved, and adjusting the pH value of the solution to 3.0-6.0;
adding activated carbon having a weight 0.2-0.02% of the total amount to the solution, mixing, stirring at room temperature, filtering, and decarbonizing the solution;
adding water for injection into the solution until the total amount is reached, measuring the pH value of the solution and the contents of active ingredients of the solution; and
after being qualified, filtering the solution repeatedly until it is clear, before putting the solution into containers, sterilizing and packaging the solution.

Temperature (℃)
40
30
20
10
0
-10
-20
-30
-40
-50
Time (h)
0
2
4
6
8
10
12
14
16
18
20
22
24
26

Fig. 1

VWD1 A, Wavelength =332 nm (ZHSHHC\BIAO0002.D)
mAU
15.198
25.458
min

Fig. 2

VWD1 A, Wavelength =332 nm (ZHSHHC\YANG0004.D)
mAU
13.341
14.717
24.251
min

Fig. 3

| INTERNATIONAL SEARCH REPORT | International application No. PCT/CN2007/003757 |
|---|---|

A. CLASSIFICATION OF SUBJECT MATTER See extra sheet

According to International Patent Classification (IPC) or to both national classification and IPC

B. FIELDS SEARCHED

Minimum documentation searched (classification system followed by classification symbols)

IPC A61K,A61P

Documentation searched other than minimum documentation to the extent that such documents are included in the fields searched

Electronic data base consulted during the international search (name of data base and, where practicable, search terms used)

CPRS, EPODOC, WPI, PAJ, CNKI (forsythoside, injection)

C. DOCUMENTS CONSIDERED TO BE RELEVANT

| Category* | Citation of document, with indication, where appropriate, of the relevant passages | Relevant to claim No. |
|---|---|---|
| X | CN1069545A(LIHong), 15 Aug. 2001 (15.08.2001), Claim 1 | 1-3, 5 |
| A | CN1069545A(LIHong), 15 Aug. 2001 (15.08.2001), Claim 1 | 4, 6-10 |
| A | JP2002173608A(SANE-N), 21 Jun. 2002 (21.06.2002), Abstract | 1-10 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 19 Mar.2008 (19.03.2008) | **10 Apr. 2008 (10.04.2008)** |

Name and mailing address of the ISA/CN
The State Intellectual Property Office, the P.R.China
6 Xitucheng Rd., Jimen Bridge, Haidian District, Beijing, China
100088
Facsimile No. 86-10-62019451

Authorized officer
ZHAI, Yu
Telephone No. (86-10)62411066

Form PCT/ISA/210 (second sheet) (April 2007)

**INTERNATIONAL SEARCH REPORT**
Information on patent family members

International application No.
PCT/CN2007/003757

| Patent Documents referred in the Report | Publication Date | Patent Family | Publication Date |
|---|---|---|---|
| CN1069545A | 15 Aug. 2001 (15.08.2001) | none | |
| JP2002173608A | 21 Jun.2002 (21.06.2002) | none | |

Form PCT/ISA/210 (patent family annex) (April 2007)

## INTERNATIONAL SEARCH REPORT

International application No.
PCT/CN2007/003757

CLASSIFICATION OF SUBJECT MATTER

A61K31/702(2006.01)i

A61K9/08(2006.01)i

A61K9/14(2006.01)i

A61P29/00(2006.01)i

A61P31/02(2006.01)i

Form PCT/ISA/210 (extra sheet) (April 2007)